# EUROPEAN PATENT SPECIFICATION

(11) **EP 3 461 788 B1**
(45) Date of publication and mention of the grant of the patent: **12.10.2022**
(21) Application number: 18173213.2
(22) Date of filing: 02.03.2017
(51) Int. Cl.: B82Y 5/00, A61L 27/54, A61L 27/18, A61L 27/36, A61L 27/48, A61L 31/00, A61L 31/06, A61L 15/26, A61L 15/40, A61L 17/10, A61L 31/16, A61L 17/00

(54) **HYBRID HONEY NANOFIBERS**
HYBRIDE HONIGNANOFASERN
NANOFIBRES HYBRIDES COMPRENANT DU MIEL

(30) Priority: 08.03.2016 ES 201600173
(43) Date of publication of application: 03.04.2019
(62) Divisional of application: 17762582.9
(73) Proprietor: Universidad de Las Palmas de Gran Canaria, 35001 Las Palmas de Gran Canaria (ES)
(72) Inventor: MONZÓN MAYOR, Maximina, 35016 Las Palmas de Gran Canaria (ES); ROMERO ALEMÁN, María del Mar, 35016 Las Palmas de Gran Canaria (ES); HERNÁNDEZ RODRÍGUEZ, José Enrique, 35016 Las Palmas de Gran Canaria (ES); PÉREZ GALVÁN, José Manuel, 35016 Las Palmas de Gran Canaria (ES)
(74) Representative: ABG Intellectual Property Law, S.L.

(56) References cited:
- WO-A1-2015/157485
- WO-A1-2015/183228
- GB-A- 2 484 319

## Description

### Field of the Invention

The present invention belongs to the field of tissue engineering. In particular, it relates to the application of hybrid honey nanofibers to nerve regeneration.

### Background of the Invention

Nerve tissue controls the homeostasis of all organs and systems of the body. Proper nerve tissue regeneration contributes to the functional regeneration of other tissues forming different organs. After traumatic injuries, successful axonal regrowth both in the central nervous system (CNS) and in large gaps between peripheral nerve endings and functional target tissue reinnervation as a result of injuries in the CNS, peripheral nerves, or local wounds (e.g., skin wounds) constitutes a challenge in the field of regenerative biomedicine today. Peripheral nerves present spontaneous regrowth capacity provided that contact between the nerve endings is restored since the distal nerve ending Schwann cells provide a favorable microenvironment. However, the structure and function of regenerated nerves differ from normal health conditions. Furthermore, target organ reinnervation is usually clinically disappointing, with significant and persistent functional deficits.

Up until now, the main method for peripheral nerve regeneration consists of replacing the damaged region with autologous and heterologous tissue transplants. However, these tissue transplants present significant limitations, such as the limited availability of autologous tissue transplants and the possibility of immune rejection of said transplants. The use of natural and synthetic materials which reproduce the natural micrometric and nanometric organization of the extracellular matrix of healthy tissues and provide an optimal microenvironment for cell adhesion, growth, proliferation, and differentiation has been proposed as an alternative.

The usefulness of synthetic polymers such as poly-3-hydroxybutyrate-co-3-hydroxyvalerate (PHBV) and poly-L-lactic acid (PLLA) in axonal regrowth is recognized in the literature [PLLA (Corey et al., J. Biomed. Mater Res. A 2007, 83(3)636-645; Wang et al., J. Neural Eng. 2009, 6(1), 016001), PHBV (Masaeli et al., 2013, PLoS One 8(2) e57157), Prabhakaran et al., 2013, Biotechnol. Bioeng.110(10)2775-84)]. Furthermore, I Uslu et al. (Hacettepe J. Biol. & Chem., 2010, 38(1)) disclose the use of hybrid Aloe vera nanofibers with synthetic polyvinyl alcohol/polyvinylpyrrolidone/polyethylene glycol polymers for wound dressing. Gupta et al (J. Biomater. Tissue Eng., 2013, 3(5) 503-11) disclose hybrid Aloe vera nanofibers, polyvinyl alcohol, polyethylene oxide, and carboxymethyl cellulose. Jithendra et al. (ACS Appl. Matter. Interfaces, 2013, 5, 7291-8) disclose collagen, chitosan, and Aloe vera nanofibers for tissue engineering. Shanmugavel et al. (J. Biomatter. Appl., 2013, 29(1) 46-58) disclose silk fibroin, caprolactone, and Aloe vera nanofibers for bone tissue engineering. Sungaya et al. 2014 (Iran Polym J., 23, 237-248) disclose silk fibroin, hydroxyapatite, and Aloe vera nanofibers for ossification. Furthermore, Wang and Ji-Huan disclose the production of hybrid honey and polyvinyl alcohol (PVA) nanofibers (Thermal Science 2013, 17:1549-1550). Maleki et al. propose the use of hybrid honey and PVA nanofibers as a wound dressing (J. Appl. Polym. Sci 2013, 127:4086-4092). Arslan et al. describe the production of hybrid honey and polyethylene terephthalate (PET) nanofibers and the potential use thereof as a wound dressing (J. Biomater. Sci. Polym. Ed. 2014, 25(10):999-1012). Sarhan et al. publish the production of hybrid honey, PVA, and chitosan nanofibers for use in tissue engineering and as a wound dressing (Material Science and Engineering C 2016, 67:276-284; Applied Materials and Interfaces 2016 8:6379-6390). GB2484319 describes electrospun nanofibers comprising honey and biocompatible synthetic polymeric material for wound dressings and tissue engineering. WO 2015/183228 discloses nanofibres comprising a mixture of polyvinyl alcohol and natural honey for use as cover for wounds and ambustions. WO2015/157485 discloses a device for promoting healing at a connection site between tubular biologic structures comprises a porous construct of nanofibers spun from a biocompatible material. None of these documents mentions structures that are efficient in nerve tissue growth and regeneration.

There is therefore a need in the state of the art to attain new structures that are more efficient in nerve regeneration, particularly in functional target tissue, peripheral nerve, or local wound reinnervation.

### Description of the Invention

The object of the present invention is to provide structures which allow nerve reconnection after peripheral nerve axotomy and to improve sensory recovery from injuries (e.g., burns, ulcers, surgical incisions, etc.) in sensory organs such as the skin, among others. The hybrid nanofibers provided in the present invention promote nerve regeneration, additionally acting as a structural support for nerve tissue growth. Furthermore, the biocompatibility of the hybrid nanofibers of the invention prevents rejection by the body.

In a first aspect, the invention relates to hybrid nanofibers comprising
a mixture of a honey and a synthetic polymer,
wherein the synthetic polymer is selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polyvinyl alcohol (PVA), polylactide-co-glycolide (PLGA), polyhydroxyalkanoates (PHAs), polypropylene carbonate (PPC), and derived mixtures, and wherein said nanofibers comprise
   - between 5 and 10% by weight of a honey; and
   - 10% by weight of the synthetic polymer.

In a second aspect, the invention relates to a method for producing the above hybrid nanofibers, wherein the method comprises:
a) preparing a solution of a honey in a solvent selected from hexafluoride-2-propanol(HFIP), polyvinyl alcohol (PVA), 1% acetic acid, or trifluoroacetic acid (TFA),
b) mixing the solution of honey of step a) with a synthetic polymer selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polylactide-co-glycolide (PLGA), polyhydroxyalkanoates (PHAs), polypropylene carbonate (PPC), and derived mixtures to form a hybrid polymer solution, and
c) injecting the mixture of step b) into electrospinning equipment for producing hybrid honey nanofibers by electrospinning.

In one aspect, the invention relates to the hybrid nanofibers comprising a mixture of a honey and a synthetic polymer,
wherein the synthetic polymer is selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polylactide-co-glycolide (PLGA), polyhydroxyalkanoates (PHAs), polypropylene carbonate (PPC), and derived mixtures,
wherein the hybrid nanofibers comprise between 5 and 10% by weight of a honey and 10% by weight of the synthetic polymer,
for use as a medicinal product.

In another additional aspect, the invention relates to the use of the hybrid nanofibers comprising a mixture of a honey and a synthetic polymer,
wherein the synthetic polymer is selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polyvinyl alcohol (PVA), polylactide-co-glycolide (PLGA), polyhydroxyalkanoates (PHAs), polypropylene carbonate (PPC), and derived mixtures,
wherein the hybrid nanofibers comprise between 5 and 10% by weight of a honey and 10% by weight of the synthetic polymer,
for use in nerve tissue regeneration; preferably wherein the nerve tissue regeneration takes place by promoting the growth, proliferation, or differentiation of any cell type located in any tissue, organ, or organ system where the nerve tissue is present. In particular, the regeneration or growth of the nerve tissue in the presence of the hybrid nanofibers of the invention occurs without requiring the presence of other additives or growth factors promoting the growth of the nerve tissue.

Furthermore, the invention also relates to the use of the hybrid nanofibers comprising a mixture of a honey and a synthetic polymer, wherein the synthetic polymer is selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polyvinyl alcohol (PVA), polylactide-co-glycolide (PLGA), polyhydroxyalkanoates (PHAs), polypropylene carbonate (PPC), and derived mixtures;
wherein the hybrid nanofibers comprise between 5 and 10% by weight of a honey and 10% by weight of the synthetic polymer, for tissue engineering applications.

### Description of the Drawings

The drawings included in the description illustrate particular embodiments of the present invention. Furthermore, in combination with the text of the description, the drawings are used to explain the principles on which the invention is based.

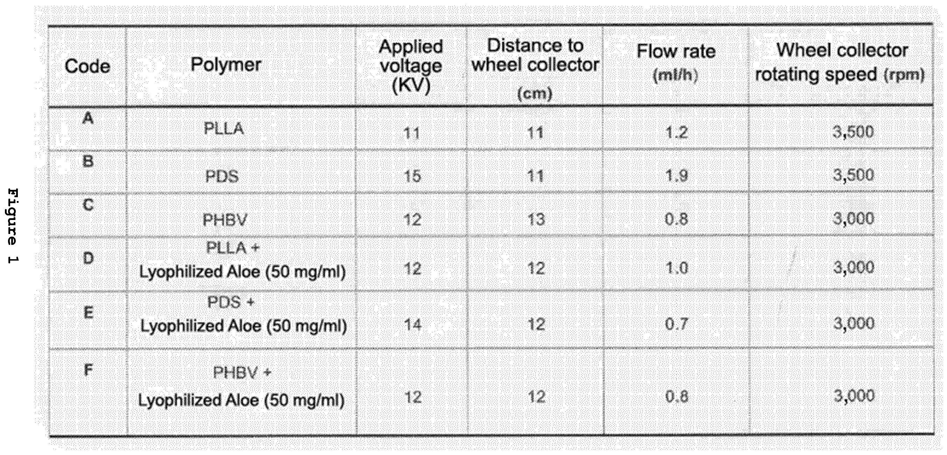

Figure 1 shows a table listing the particular conditions used for the formation of synthetic polymer nanofibers and hybrid Aloe vera nanofibers (not according to the invention) by electrospinning.
Figure 2 shows the scanning electron microscopy micrograph of electrospun polymers: A. PLLA. B. PLLA + Aloe vera, C. PDS, D. PDS + Aloe vera; E. PHBV; F. PHBV + Aloe vera. Scales (A-F): 60 µm. Scales in the insert image (A-F): 6 µm.

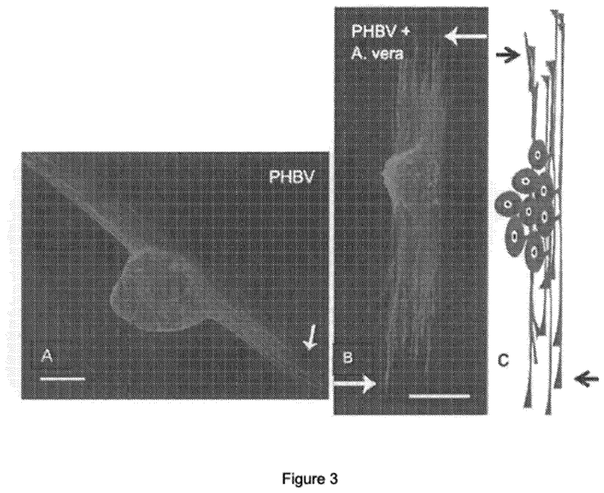

Figure 3 shows the immunofluorescence images of rat dorsal root ganglion (DRG) explant neurons cultured in the presence of aligned synthetic PHBV polymer nanofibers (Figure 3A), and in the presence of the aligned hybrid PHBV and Aloe vera nanofibers (Figure 3B) (not according to the invention) . . Figure 3C is a representative diagram of Figures 3A and 3B, showing the neuronal bodies (spherical structures) and their nerve projections following the path of the aligned nanofibers. The arrows in Figures 3A, 3B, and 3C indicate the location of the growth cones. Scale (Figures 3A and 3B): 200 µm.
Figure 4 shows the scanning electron microscopy micrograph of unaligned nanofibers oriented in different directions with respect to one another: A. Honey + PHVB; B. Aloe vera + PHBV, and C. PHBV. Scale (Figures 4A, 4B, and 4C): 20 µm.
Figure 5A shows an immunofluorescence image (confocal microscope) of rat DRG explant neurons cultured in the presence of unaligned nanofibers of Figure 4A. Figure 5B is a representative diagram of Figure 5A showing the neuronal bodies (spherical central structure) and the growth of their neurites following the path of the unaligned hybrid nanofibers.

### Detailed Description of the Invention

### Hybrid honey and synthetic polymer nanofibers

The hybrid nanofibers comprise a mixture of a honey made by bees and a synthetic polymer, wherein the synthetic polymer is selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polyvinyl alcohol (PVA), polylactide-co-glycolide (PLGA), polyhydroxyalkanoates (PHAs), polypropylene carbonate (PPC), and derived mixtures. In a particular embodiment, the hybrid honey nanofibers may comprise a mixture of the components of honey and medical grade biodegradable thermoplastic polyurethanes such as Z3A1 or Z9A1 (Biomer Technology LTD), DegraPol^{®} (a polyester-urethane formed by two polyester diols bound by an isocyanate group), or Desmopan^{®} 9370A (an ether having four carbon atoms) from Bayer.

In a preferred embodiment, the hybrid honey and synthetic polymer nanofibers comprise a mixture of the components of a honey made by bees and a synthetic polymer selected from poly-3-hydroxybutyrate-co-3-hydroxyvalerate (PHBV), poly-L-lactic acid (PLLA), polydioxanone (PDS), and derived mixtures

Bees produce honey from flower nectar, from secretions from living parts of plants, or from the exudates produced by plant-sucking insects. Honey made by bees is directly extracted from honeycomb and kept in a sterile container at 8°C before the formation of the hybrid nanofibers.

For the preparation of the hybrid honey nanofibers, the honey used can be any type of honey. Particularly, the honey used can be floral honey such as monofloral honey, multifloral honey, honey from a mountain range, a mountain, or a desert. The honey of the hybrid nanofibers can also be honeydew honey, honeydew, dew honey, or forest honey.

Generally, the composition of honey known in the state of the art comprises the following components:
- 14-22% by weight of water,
- 28-44% by weight of fructose,
- 22-40% by weight of glucose,
- 0.2-7% by weight of sucrose,
- 2-16% by weight of maltose,
- 0.1-8% by weight of other sugars,
- 0.2-2% by weight of proteins and amino acids,
- 0.5-1% by weight of vitamins, enzymes, hormones, and organic acids,
- 0.5-1% by weight of minerals, and
- 0.2-1% by weight of ash.

In a particular embodiment, the composition of the honey comprises:
- 18% by weight of water,
- 38% by weight of fructose,
- 31% by weight of glucose,
- 1% by weight of sucrose,
- 7.5% by weight of maltose, and
- 5% by weight of other sugars.

The honey used by the inventors comes from a local beekeeper in Gran Canaria who is registered in the General Health Registry for Food Companies and Foodstuffs (*Registro General Sanitario de Empresas Alimentarias y Alimentos* - RGSEAA): Maria del Rosario Cazorla López. RGSEAA No.: 23.03229/GC.

The hybrid honey nanofibers comprising a mixture of the components of a honey and a synthetic polymer contain between 5 and 10% by weight of a honey and 10% by weight of a synthetic polymer selected from poly-3-hydroxybutyrate-co-3-hydroxyvalerate (PHBV), poly-L-lactic acid (PLLA), polydioxanone (PDS), polycaprolactone (PCL), polyvinyl alcohol (PVA), polylactide-co-glycolide (PLGA), polyhydroxyalkanoates (PHAs), polypropylene carbonate (PPC), and medical grade biodegradable thermoplastic polyurethanes such as Z3A1 or Z9A1 (Biomer Technology LTD), DegraPol^{®} (a polyester-urethane formed by two polyester diols bound by an isocyanate group), Desmopan^{®} 9370A (an ether having four carbon atoms) from Bayer, or derived mixtures. The hybrid nanofibers preferably contain between 6 and 8% by weight of a honey and 10% by weight of a synthetic polymer.

In a particular embodiment, the ratio by weight of the components of honey and synthetic polymer in the hybrid nanofibers of the invention is comprised between 33:67 and 50:50. The ratio by weight of the components of honey and synthetic polymer is preferably comprised between 35:65 and 48:52, preferably between 37:62 and 46:54, more preferably between 40:60 and 45:55. In a preferred embodiment, the ratio by weight of honey/synthetic polymer is comprised between 42:58 and 44:56.

In a particular embodiment, the hybrid honey and synthetic polymer nanofibers of the invention have an approximate diameter between 0.3 and 1.5 microns, preferably between 0.5 and 1.3, more preferably between 0.7 and 1.2 In another embodiment, the hybrid honey and synthetic polymer nanofibers of the invention have an approximate diameter between 0.8 and 1.1 microns, preferably between 0.9 and 1 micron. The hybrid honey nanofibers, like the hybrid Aloe vera nanofibers, act as a support for the adherence and guidance of nerve cells in axonal growth during a process of regeneration and healing of any tissue or organ having nerve structures. In particular, the hybrid honey nanofibers of the present invention act as a support for axonal regeneration in the peripheral nervous system (PNS) and central nervous system (CNS).

In another embodiment, the hybrid honey nanofibers of the invention can be aligned, i.e., oriented in one and the same direction, or disorganized, i.e., oriented in different directions with respect to one another. The hybrid honey nanofibers of the invention are preferably aligned.

In a particular embodiment, the hybrid honey nanofibers comprising a mixture of components of a honey and poly-3-hydroxybutyrate-co-3-hydroxyvalerate (PHBV) have an average diameter between 0.8 and 1.5 microns and are aligned in a specific direction or oriented in different directions with respect to one another.

In another particular embodiment, the hybrid honey nanofibers comprising a mixture of components of a honey and poly-L-lactic acid (PLLA) have an average diameter between 0.8 and 1.5 microns and are aligned in a specific direction or oriented in different directions with respect to one another.

In another particular embodiment, the hybrid honey nanofibers comprising a mixture of components of a honey and polydioxanone (PDS) have an average diameter between 0.8 and 1.5 microns and are aligned in a specific direction or oriented in different directions with respect to one another.

### Method of producing the hybrid honey and synthetic polymer nanofibers

The hybrid honey and synthetic polymer nanofibers of the invention can be obtained by means of a manufacturing method similar to that used for producing the hybrid Aloe vera and synthetic polymer fibers. The method for producing the hybrid honey nanofibers comprises:
a) preparing a solution of a honey in a solvent selected from hexafluoride-2-propanol, polyvinyl alcohol (PVA), 1% acetic acid, and trifluoroacetic acid (TFA).
b) mixing the solution of honey of step a) with a synthetic polymer selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polylactide-co-glycolide (PLGA), polyhydroxyalkanoates (PHAs), polypropylene carbonate (PPC), medical grade biodegradable thermoplastic polyurethanes and derived mixtures, to form a hybrid polymer solution, and
c) injecting the mixture of step b) into electrospinning equipment to produce hybrid honey nanofibers by electrospinning.

According to the method described above, a solution of honey in hexafluoride-2-propanol (HFIP), polyvinyl alcohol (PVA), 1% acetic acid, and trifluoroacetic acid (TFA) is prepared in step a). Said solution usually has a concentration of 50 to 100 mg/ml of honey

In step b) of the method described above, the solution of honey of step a) is mixed with a synthetic polymer selected from poly-3-hydroxybutyrate-co-3-hydroxyvalerate (PHBV), poly-L-lactic acid (PLLA), polydioxanone (PDS), polycaprolactone (PCL), polylactide-co-glycolide (PLGA), polyhydroxyalkanoates (PHAs), polypropylene carbonate (PPC), medical grade biodegradable thermoplastic polyurethanes such as Z3A1 or Z9A1, DegraPol^{®}, or Desmopan^{®} 9370A, and derived mixtures to form a hybrid polymer solution.

In a particular embodiment, the ratio by weight of the honey and the synthetic polymer in the hybrid polymer solution is comprised between 33:67 and 50:50 (honey:synthetic polymer). The ratio by weight of honey/synthetic polymer in the hybrid polymer solution is preferably comprised between 35:65 and 48:52, preferably between 37:62 and 46:54, more preferably between 40:60 and 45:55. In a preferred embodiment, the ratio by weight of honey/synthetic polymer in the hybrid polymer solution is comprised between 42:56 and 44:56.

In step c) of the method of the invention, the mixture of step b) is injected into electrospinning equipment to produce hybrid honey nanofibers by electrospinning. In particular, the mixture is loaded into a syringe pump connected to an electrode. The solution is driven at a flow rate between 2 and 3 ml/h, preferably at a flow rate between 2.5 and 3 ml/h, more preferably between 2.5 and 2.8 ml/h; and applying a potential of 7 to 11 kV, preferably 8 to 10 kV, more preferably 8 to 9 kV, from the syringe to a rotating wheel collector. The rotary wheel collector can rotate at a speed between 2000 and 4000 rpm, preferably 3000 rpm. The wheel collector can likewise rotate at other speeds. In particular, when the rotating speed is comprised between 100 and 300 rpm, preferably 200 rpm, the nanofibers obtained are not aligned and are oriented in different directions with respect to one another. The rotary wheel collector is located at a distance of 8 to 12 cm from the syringe pump, preferably at a distance of 9 to 11 cm from the syringe pump, more preferably at a distance of 10 cm from the syringe pump. The conditions used in electrospinning are environmental conditions with a humidity of 60-70% and a temperature of 20-25°C.

The hybrid nanofibers obtained by means of the method described above comprise a mixture of the components of a honey and a synthetic polymer selected from poly-3-hydroxybutyrate-co-3-hydroxyvalerate (PHBV), poly-L-lactic acid (PLLA), and polydioxanone (PDS), polycaprolactone (PCL), polylactide-co-glycolide (PLGA), polyhydroxyalkanoates (PHAs), polypropylene carbonate (PPC), medical grade biodegradable thermoplastic polyurethanes such as Z3A1 or Z9A1, DegraPol^{®}, or Desmopan^{®} 9370A, and derived mixtures, wherein the hybrid nanofibers have an average diameter comprised between 0.3 and 1.5 microns. In a preferred embodiment, the hybrid nanofibers obtained by means of the method described above comprise a mixture of honey and a synthetic polymer selected from poly-3-hydroxybutyrate-co-3-hydroxyvalerate (PHBV), poly-L-lactic acid (PLLA), polydioxanone (PDS), and derived mixtures.

The hybrid honey nanofibers can be used for tissue engineering applications, particularly for nerve tissue regeneration. In this sense, the hybrid honey nanofibers can be used for nerve tissue regeneration in nerve connection prostheses, dressings, sutures in skin wounds, and surgical meshes. The invention therefore relates to nerve connection prostheses, dressings, sutures in skin wounds, or surgical meshes comprising the hybrid honey nanofibers that have been described. Likewise, the hybrid honey fibers can be used in bandages and dressings to cover wounds.

The hybrid honey nanofibers can also be used for regenerating other tissues, such as tendon, ligament and bone tissues.

The invention relates to the hybrid honey nanofibers of the invention for use as a medicinal product. Likewise, the invention relates to the hybrid honey nanofibers for use in the treatment for nerve tissue regeneration in any vertebrate, or for the growth, proliferation, or differentiation of any cell type located in any tissue, organ, or organ system where the nerve tissue is present.

### Examples

### 1. Preparing aligned hybrid Aloe vera and synthetic polymer fibers (not according to the invention)

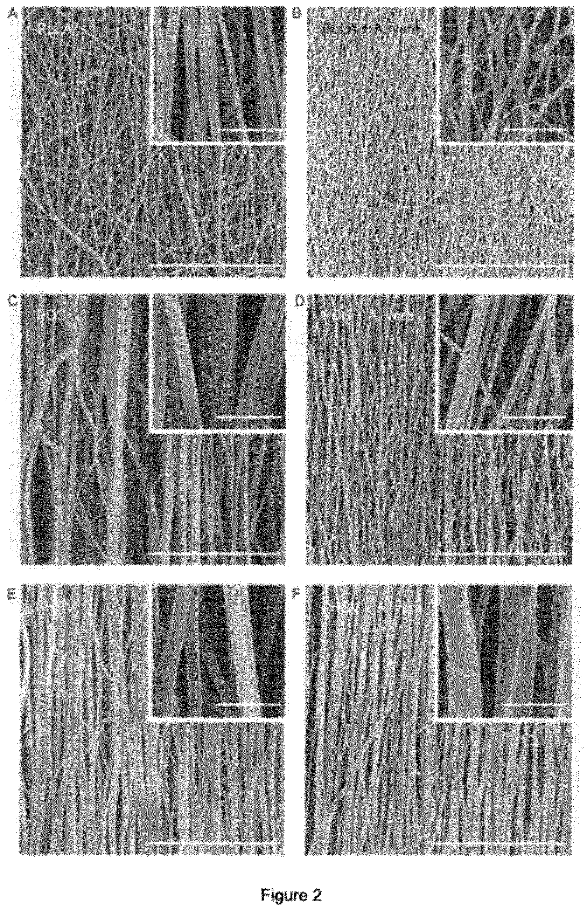

First, a solution of 25-50 mg/ml of Aloe vera (Prod. No. 001, Laboratorios Luciano Reverón e hijos S.L., Tenerife) in hexafluoro-2-propanol (HFIP) was prepared. To obtain the hybrid solution (AV/PHBV), 10-12% (w/w) of PHBV (Sigma-Aldrich, Prod. No. 403121) was added to the preceding solution. To obtain aligned nanofibers by means of the electrospinning technique, the hybrid polymer solution was loaded into a syringe pump (Hardvard Apparatus PhD Ultra) with a needle (G20, 0.9 mm in diameter) the tip of which was connected to an electrode (spinnerette). 12-15 KV were applied with a high-voltage source (Spellman 60N300) while the syringe pump discharged the solution (flow rate of 0.9 ml/h) under environmental conditions with a humidity of 60-65% and a temperature of 22-25°C towards a target wheel (90 mm in diameter and 12 mm thick) located 12 cm form the electrode. Bundles of aligned nanofibers were collected on the target wheel rotating at 3000 rpm. Figure 2F shows SEM micrographs of the nanofibers that were obtained.

Additionally, aligned hybrid fibers of the invention consisting of PLLA and Aloe vera, PDS and Aloe vera and PHVB and Aloe vera, and synthetic PLLA, PDS, and PHBV polymer fibers were prepared following the same method. Figures 2A to 2E show SEM micrographs of the aligned fibers obtained from the synthetic PLLA polymer (Figure 2A), PDS polymer (Figure 2C), and PHBV polymer (Figure 2E); as well as from the hybrid Aloe vera fibers of the invention consisting of PLLA + Aloe vera (Figure 2B), PDS + Aloe vera (Figure 2D) following the same method that has been described.

### 2. Neuritic growth comparative assays

Comparative assays were performed using newborn rat (Sprague Dawley) dorsal root ganglion (DRG) explant culture in a) a standard culture medium [DMEM/F12 (1:1)] to establish the control conditions, and b) in DMEM/F12 (1:1) containing the invented hybrid nanometric matrix (AV PHBV) and other pure reference substances (PHBV and PLLA) as the experimental substrate for neuritic growth. The rat DRG explants were incubated in an oven at 37°C and under a 5% CO₂ atmosphere for 6 days, and the culture medium was changed after 3 days.

After the incubation period has elapsed, the rat DRG explants were fixed with a 4% paraformaldehyde solution in phosphate-buffered saline and immunolabeled with antibodies specific for neuron identification. Digital images were then taken in a fluorescence microscope equipped with an image capturing system. The images were processed for statistical analysis.

A statistical analysis was performed in each of the polymers, the positive area ratios being summarized as medians and interquartile ranges in each of the treatment groups. The statistical analyses demonstrated that the highest neuritic growth rate occurred with the aligned hybrid AV/PHBV nanofibers in comparison with the absence of aloe in aligned pure PHBV nanofibers (p<0.001) and pure PLLA nanofibers (p=0.049).

### 3. Preparing hybrid fibers oriented in different directions with respect to one another (not according to the invention)

First, a solution of 50 mg/ml of Aloe vera (Prod. No. 001 , Laboratories Luciano Reverón e hijos S.L , Tenerife) in hexafluoro-2-propanol (HFIP) was prepared. The Aloe vera of the solution was lyophilized and filtered Aloe vera having a size of 22 µm. To obtain the hybrid solution (AV/PHBV), 10% by weight of PHBV (Sigma-Aldrich, Prod. No. 403121) was added to the preceding solution (400 mg of PHBV in 3,600 mg HFIP+ Aloe vera).

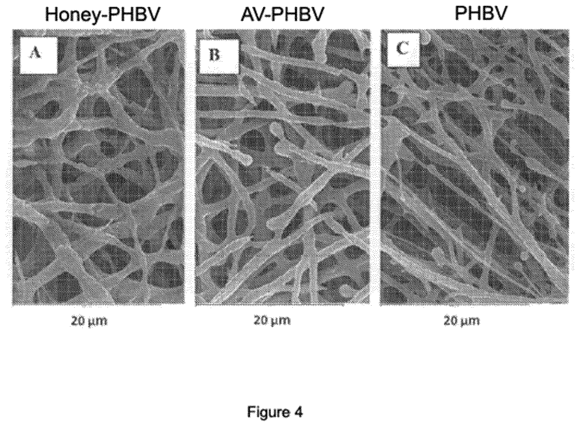

To obtain the nanofibers by means of the electrospinning technique, the hybrid polymer solution was loaded into a syringe pump (Hardvard Apparatus PhD Ultra) with a needle (16G) the tip of which was connected to an electrode (spinnerette). 9 KV were applied with a high-voltage source (Spellman 60N300) while the syringe pump discharged the solution (flow rate of 2.75 ml/h) under environmental conditions with a humidity of 60-65% and a temperature of 25-26°C towards a target wheel (90 mm in diameter/8 mm thick) located 10 cm from the electrode. Unaligned nanofibers oriented in different directions with respect to one another were collected on the target wheel rotating at 200 rpm. Figure 4B shows SEM micrographs of the hybrid Aloe vera nanofibers that were obtained. The diameters of the obtained nanofibers were measured from the SEM images using the Image J computer program (NIH, USA). The average measured diameter of the hybrid Aloe vera nanofibers obtained is 1.0241 µm, the diameter of the unaligned hybrid nanofibers that were obtained ranging between 0.9854 µm and 1.0628 µm.

The same method was followed to manufacture unaligned hybrid honey fibers oriented in different directions with respect to one another, but using honey from a local beekeeper in Gran Canaria who is registered in the General Health Registry for Food Companies and Foodstuffs (RGSEAA): Maria del Rosario Cazorla Lopez, RGSEAA No.: 23.03229/GC, instead of Aloe vera. Figure 4A shows SEM micrographs of the hybrid honey and PHBV nanofibers that were obtained. The diameters of the obtained nanofibers were measured from the SEM images using the Image J computer program (NIH, USA). The average measured diameter of the obtained hybrid honey nanofibers is 1.2528 µm, the diameter of the unaligned hybrid nanofibers that were obtained ranging between 0.9801 µm and 1.5255 µm.

Unaligned PHVB nanofibers were prepared for comparison. In this case, 12% by weight of PHBV (Sigma-Aldrich, Prod. No. 403121) in hexafluoro-2-propanol (HFIP) was added to obtain a solution with a concentration of 10% by weight of PHBV. The hybrid polymer solution was loaded into a syringe pump (Hardvard Apparatus PhD Ultra) with a needle (16G) the tip of which was connected to an electrode (spinnerette). 12 KV were applied with a high-voltage source (Spellman 60N300) while the syringe pump discharged the solution (flow rate of 1 ml/h) under environmental conditions with a humidity of 60-65% and a temperature of 25-26°C towards a target wheel (90 mm in diameter/8 mm thick) located 12 cm from the electrode. Nanofibers oriented in different directions with respect to one another were collected on the target wheel rotating at 200 rpm. Figure 4C shows SEM micrographs of the nanofibers that were obtained. The diameters of the obtained nanofibers were measured from the SEM images using the Image J computer program (NIH, USA). The average measured diameter of the unaligned PHVB nanofibers is 0.9130 µm, the diameter of the unaligned PHVB nanofibers obtained ranging between 0.7803 µm and 1.0457 µm.

### 4. In vitro neuritic growth comparative assays

Comparative assays were performed using newborn rat (Sprague Dawley) dorsal root ganglion (DRG) explant culture in a standard culture medium [DMEM/F12 (1:1)], the nanometric matrix containing the unaligned honey/PHBV, AV/PHBV, and PHBV fibers of Example 3 as an experimental substrate for neuritic growth. The rat DRG explants were incubated in an oven at 37°C and under a 5% CO₂ atmosphere for 6 days, and the culture medium was changed after 3 days.

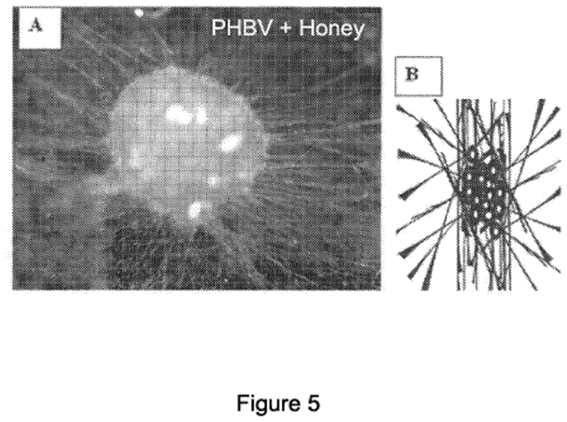

After the incubation period has elapsed, the rat DRG explants were fixed with a 4% paraformaldehyde solution in phosphate-buffered saline and immunolabeled with specific antibodies for the identification of neurons, Schwann cells, and cell nuclei. Digital images were then taken in a fluorescence microscope equipped with an image capturing system. Greater neuritic growth was observed in the presence of hybrid honey/PHBV and AV/PHBV nanofibers than in the presence of PHBV nanofibers. Figure 5A shows the immunofluorescence image of the growth of many neurites in all directions from the rat DRG explant (spherical central structure in the image). The growing neurites follow the path of the unaligned hybrid nanofibers, as depicted in the diagram of Figure 5B.

### 5. In vivo skin wound healing comparative assays

With the required authorization from the Ethics Committee on Animal Experimentation, the murine model for wound (8 mm in diameter) repair (subcutaneous ring) was used to mimic healing by second intention in humans (chronic skin ulcers). A comparative study was performed on the effect of the nanometric matrix containing the unaligned hybrid honey/PHBV, AV/PHBV, and PHBV nanofibers of Example 3 with daily treatments with natural honey and commercial dressing (Mepilex border). The mentioned matrices were applied on the wound bed with no additional care other than protecting same with a surgical adhesive. After 8 days, samples were taken for microbiological cultures (*Staphylococcus aureus* and *E. coli*). The preliminary data indicates that wound closure occurred earlier in groups treated with the hybrid matrices: honey/PHBV (12.5 days on average), AV/PHBV (13 days on average) and daily treatments with natural honey (13.3 days on average) compared to groups treated with pure PHBV matrix (14.3 days on average) or the commercial dressing (15 days on average). The bacteriological analyses were negative.

## Claims

1. Hybrid nanofibers comprising
a mixture of a honey and a synthetic polymer,
wherein the synthetic polymer is selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polyvinyl alcohol (PVA), polylactide-co-glycolide (PLGA), polyhydroxyalkanoates (PHAs), polypropylene carbonate (PPC), and derived mixtures; and
wherein said nanofibers comprise
- between 5 and 10% by weight of a honey; and
- 10% by weight of the synthetic polymer.

2. The hybrid nanofibers according to claim 1, wherein the synthetic polymer is selected from poly-3-hydroxybutyrate-co-3-hydroxyvalerate (PHBV), poly-L-lactic acid (PLLA), polydioxanone (PDS), and derived mixtures.

3. The hybrid nanofibers according to claims 1 or 2, wherein the synthetic polymer is poly-3-hydroxybutyrate-co-3-hydroxyvalerate (PHBV).

4. The hybrid nanofibers according to any of claims 1 to 3, wherein the hybrid nanofibers contain between 6 and 8% by weight of a honey and 10% by weight of the synthetic polymer.

5. The hybrid nanofibers according to any of claims 1 to 3, wherein the ratio by weight of honey and synthetic polymer in the hybrid nanofibers is comprised between 33:67 and 50:50.

6. The hybrid nanofibers according to any of claims 1 to 5, wherein the average diameter of the hybrid nanofibers is comprised between 0.3 and 1.5 microns.

7. The hybrid nanofibers according to any of claims 1 to 6, wherein said nanofibers are aligned in a specific direction or oriented in different directions with respect to one another.

8. The hybrid nanofibers according to any of claims 1 to 7,
wherein said nanofibers comprise a mixture of a honey and a synthetic polymer selected from poly-3-hydroxybutyrate-co-3-hydroxyvalerate (PHBV), poly-L-lactic acid (PLLA) or polydioxanone (PDS),
wherein said hybrid nanofibers have an average diameter between 0.8 and 1.5 microns, and
wherein the nanofibers are aligned in a specific direction or oriented in different directions with respect to one another.

9. A method for producing hybrid nanofibers according to claims 1 to 8, wherein the method comprises:
a) preparing a solution of a honey in a solvent selected from hexafluoride-2-propanol (HFIP), polyvinyl alcohol (PVA), 1% acetic acid, or trifluoroacetic acid (TFA),
b) mixing the solution of honey of step a) with a synthetic polymer selected from poly-3-hydroxybutyrate-co-3-hydroxyvalerate (PHBV), poly-L-lactic acid (PLLA), polydioxanone (PDS), medical grade biodegradable thermoplastic polyurethanes, polycaprolactone (PCL), polylactide-co-glycolide (PLGA), polyhydroxyalkanoates (PHAs), polypropylene carbonate (PPC), and derived mixtures to form a hybrid polymer solution, and
c) injecting the mixture of step b) into electrospinning equipment for producing hybrid honey nanofibers by electrospinning.

10. The method according to claim 9, wherein in step b) the ratio by weight of the honey and the synthetic polymer is comprised between 33:67 and 50:50.

11. Hybrid nanofibers according to claim 1, for use as a medicinal product.

12. Hybrid nanofibers according to claim 1 for use in nerve tissue regeneration.

13. Hybrid nanofibers for use according to claim 12, wherein the nerve tissue regeneration takes place by promoting the growth, proliferation, or differentiation of any cell type located in any tissue, organ or system where the nerve tissue is present.

14. Hybrid according to claim 1 for tissue engineering applications; preferably for nerve tissue engineering applications.

## Patentansprüche

1. Hybride Nanofasern umfassend
eine Mischung aus einem Honig und einem synthetischen Polymer,
wobei das synthetische Polymer ausgewählt ist aus Poly-3-hydroxybutyrat-co-3-hydroxyvalerat (PHBV), Poly-L-Milchsäure (PLLA), Polydioxanon (PDS), biologisch abbaubaren thermoplastischen Polyurethanen medizinischer Qualität, Polycaprolacton (PCL), Polyvinylalkohol (PVA), Polylactid-coglycolid (PLGA), Polyhydroxyalkanoaten (PHAs), Polypropylencarbonat (PPC) und davon abgeleiteten Mischungen; und
wobei die Nanofasern
- zwischen 5 und 10 Gew.-% eines Honigs; und
- 10 Gew.-% des synthetischen Polymers umfassen.

2. Hybride Nanofasern nach Anspruch 1, wobei das synthetische Polymer ausgewählt ist aus Poly-3-hydroxybutyrat-co-3-hydroxyvalerat (PHBV), Poly-L-Milchsäure (PLLA), Polydioxanon (PDS) und davon abgeleiteten Mischungen.

3. Hybride Nanofasern nach Anspruch 1 oder 2, wobei das synthetische Polymer Poly-3-hydroxybutyrat-co-3-hydroxyvalerat (PHBV) ist.

4. Hybride Nanofasern nach einem der Ansprüche 1 bis 3, wobei die hybriden Nanofasern zwischen 6 und 8 Gew.-% eines Honigs und 10 Gew.-% des synthetischen Polymers enthalten.

5. Hybride Nanofasern nach einem der Ansprüche 1 bis 3, wobei das Gewichtsverhältnis von Honig und synthetischem Polymer in den hybriden Nanofasern zwischen 33:67 und 50:50 liegt.

6. Hybride Nanofasern nach einem der Ansprüche 1 bis 5, wobei der durchschnittliche Durchmesser der hybriden Nanofasern zwischen 0,3 und 1,5 Mikrometer liegt.

7. Hybride Nanofasern nach einem der Ansprüche 1 bis 6, wobei die Nanofasern in einer bestimmten Richtung ausgerichtet oder in verschiedenen Richtungen zueinander orientiert sind.

8. Hybride Nanofasern nach einem der Ansprüche 1 bis 7,
wobei die Nanofasern eine Mischung aus einem Honig und einem synthetischen Polymer, ausgewählt aus Poly-3-hydroxybutyrat-co-3-hydroxyvalerat (PHBV), Poly-L-Milchsäure (PLLA) oder Polydioxanon (PDS), umfassen,
wobei die hybriden Nanofasern einen durchschnittlichen Durchmesser zwischen 0,8 und 1,5 Mikrometern haben, und
wobei die Nanofasern in einer bestimmten Richtung ausgerichtet oder in verschiedenen Richtungen zueinander orientiert sind.

9. Verfahren zur Herstellung von hybriden Nanofasern nach einem der Ansprüche 1 bis 8, wobei das Verfahren Folgendes umfasst:
a) Herstellen einer Lösung eines Honigs in einem Lösungsmittel, ausgewählt aus Hexafluorid-2-propanol (HFIP), Polyvinylalkohol (PVA), 1%iger Essigsäure oder Trifluoressigsäure (TFA),
b) Mischen der Lösung des Honigs aus Schritt a) mit einem synthetischen Polymer, ausgewählt aus Poly-3-hydroxybutyrat-co-3-hydroxyvalerat (PHBV), Poly-L-Milchsäure (PLLA), Polydioxanon (PDS), biologisch abbaubare thermoplastische Polyurethane medizinischer Qualität, Polycaprolacton (PCL), Polylactid-coglycolid (PLGA), Polyhydroxyalkanoate (PHAs), Polypropylencarbonat (PPC) und abgeleitete Mischungen zur Bildung einer Hybridpolymerlösung und
c) Injizieren der Mischung aus Schritt b) in eine Elektrospinnanlage zur Herstellung von hybriden Honig-Nanofasern durch Elektrospinnen.

10. Verfahren nach Anspruch 9, wobei in Schritt b) das Gewichtsverhältnis des Honigs und des synthetischen Polymers zwischen 33:67 und 50:50 liegt.

11. Hybride Nanofasern nach Anspruch 1 zur Verwendung als Medizinprodukt.

12. Hybride Nanofasern nach Anspruch 1 zur Verwendung bei der Regeneration von Nervengewebe.

13. Hybride Nanofasern zur Verwendung gemäß Anspruch 12, wobei die Regeneration von Nervengewebe durch Förderung des Wachstums, der Proliferation oder der Differenzierung eines beliebigen Zelltyps erfolgt, der sich in einem beliebigen Gewebe, Organ oder System befindet, in dem das Nervengewebe vorhanden ist.

14. Hybride Nanofasern nach Anspruch 1 für Gewebe-Engineering-Anwendungen; vorzugsweise für Nervengewebe-Engineering-Anwendungen.

## Revendications

1. Nanofibres hybrides comprenant
un mélange d'un miel et d'un polymère synthétique,
dans lesquelles le polymère synthétique est choisi parmi le poly-3-hydroxybutyrate-co-3-hydroxyvalérate (PHBV), l'acide poly-L-lactique (PLLA), la polydioxanone (PDS), les polyuréthanes thermoplastiques biodégradables de qualité médicale, le polycaprolactone (PCL), l'alcool polyvinylique (PVA), le polylactide-co-glycolide (PLGA), les polyhydroxyalcanoates (PHAs), le carbonate de polypropylène (PPC), et des mélanges dérivés ; et
dans lesquelles lesdites nanofibres comprennent
- entre 5 et 10 % en poids d'un miel ; et
- 10 % en poids du polymère synthétique.

2. Nanofibres hybrides selon la revendication 1, dans lesquelles le polymère synthétique est choisi parmi le poly-3-hydroxybutyrate-co-3-hydroxyvalérate (PHBV), l'acide poly-L-lactique (PLLA), la polydioxanone (PDS), et des mélanges dérivés.

3. Nanofibres hybrides selon l'une quelconque des revendications 1 ou 2, dans lesquelles le polymère synthétique est le poly-3-hydroxybutyrate-co-3-hydroxyvalérate (PHBV).

4. Nanofibres hybrides selon l'une quelconque des revendications 1 à 3, dans lesquelles les nanofibres hybrides contiennent entre 6 et 8 % en poids d'un miel et 10 % en poids de polymère synthétique.

5. Nanofibres hybrides selon l'une quelconque des revendications 1 à 3, dans lesquelles le ratio en poids de miel et de polymère synthétique dans les nanofibres hybrides est compris entre 33:67 et 50:50.

6. Nanofibres hybrides selon l'une quelconque des revendications 1 à 5, dans lesquelles le diamètre moyen des nanofibres hybrides est compris entre 0,3 et 1,5 microns.

7. Nanofibres hybrides selon l'une quelconque des revendications 1 à 6, dans lesquelles lesdites nanofibres sont alignées dans une direction spécifique ou orientées dans différentes directions les unes par rapport aux autres.

8. Nanofibres hybrides selon l'une quelconque des revendications 1 à 7, dans lesquelles lesdites nanofibres comprennent un mélange d'un miel et d'un polymère synthétique choisi parmi le poly-3-hydroxybutyrate-co-3-hydroxyvalérate (PHBV), l'acide poly-L-lactique (PLLA) ou la polydioxanone (PDS),
dans lesquelles lesdites nanofibres hybrides ont un diamètre moyen compris entre 0,8 et 1,5 microns, et
dans lesquelles les nanofibres sont alignées dans une direction spécifique ou orientées dans différentes directions les unes par rapport aux autres.

9. Procédé de production de nanofibres hybrides selon les revendications 1 à 8, dans lequel le procédé comprend :
a) préparer une solution d'un miel dans un solvant choisi parmi l'hexafluoro-2-propanol (HFIP), l'alcool polyvinylique (PVA), l'acide acétique à 1%, ou l'acide trifluoroacétique (TFA),
b) mélanger la solution de miel de l'étape a) avec un polymère synthétique choisi parmi le poly-3-hydroxybutyrate-co-3-hydroxyvalérate (PHBV), l'acide poly-L-lactique (PLLA), la polydioxanone (PDS), les polyuréthanes thermoplastiques biodégradables de qualité médicale, le polycaprolactone (PCL), le polylactide-co-glycolide (PLGA), des polyhydroxyalcanoates (PHAs), le carbonate de polypropylène (PPC), et des mélanges dérivés pour former une solution de polymère hybride, et
c) injecter le mélange de l'étape b) dans un équipement d'électrofilage pour produire des nanofibres de miel hybrides par électrofilage.

10. Procédé selon la revendication 9, dans lequel, à l'étape b), le ratio en poids du miel et du polymère synthétique est compris entre 33:67 et 50:50.

11. Nanofibres hybrides selon la revendication 1, pour une utilisation en tant que produit médical.

12. Nanofibres hybrides selon la revendication 1, pour une utilisation dans la régénération de tissu nerveux.

13. Nanofibres hybrides pour une utilisation selon la revendication 12, dans laquelle la régénération de tissu nerveux prend place en favorisant la croissance, la prolifération, ou la différenciation d'un type cellulaire quelconque situé dans un tissu, organe ou système quelconque où le tissu nerveux est présent.

14. Nanofibres hybrides selon la revendication 1, pour des applications en ingénierie tissulaire ; de préférence des applications en ingénierie de tissu nerveux.
